# EUROPEAN PATENT APPLICATION

(11) **EP 4 821 999 A1**
(43) Date of publication of application: **14.10.2026**
(21) Application number: 25218919.6
(22) Date of filing: 27.11.2025
(51) Int. Cl.: C09K 5/10, F24T 50/00

(54) **LIQUID COMPOSITION, GEOTHERMAL WELL COMPRISING LIQUID COMPOSITION AND METHOD FOR PREPARING LIQUID COMPOSITION**

(30) Priority: 10.04.2025 FI 20255331
(71) Applicant: VesiTekno Oy, 01740 Vantaa (FI)
(72) Inventor: Puputti, Jyrki, 01740 Vantaa (FI)
(74) Representative: Moosedog Oy

(57) **Abstract**

Disclosed is a liquid composition for transferring heat in a geothermal well, the liquid composition comprising ethanol (EtOH); and potassium formate (KFo); wherein a mixture of the ethanol (EtOH) and the potassium formate (KFo) is dissolved in a solvent.

## Description

### TECHNICAL FIELD

The present disclosure relates to liquid compositions for transferring heat in geothermal wells. Moreover, the present disclosure relates to geothermal wells comprising liquid compositions. Furthermore, the present disclosure relates to methods for preparing liquid compositions for transferring heat in geothermal wells.

### BACKGROUND

In recent times, geothermal energy has emerged as a sustainable and renewable energy source that requires extracting heat from the Earth's crust for various applications, such as power generation and heating. The extraction of the heat from the Earth's crust to the surface, requires the use of specialized liquid compositions capable of operating under extreme temperature and pressure conditions. However, maintaining a stability, thermal efficiency, and environmental compatibility of existing liquid compositions while ensuring long-term performance in harsh geothermal environments poses a significant challenge.

The existing solutions include using different heat transfer fluids, such as brines, glycols, and other chemical mixtures, which are designed to operate under the harsh geothermal environments in the geothermal wells. However, said existing solutions also have major drawbacks, such as many of the heat transfer fluids are prone to freezing at low temperatures, leading to operational inefficiencies and potential system failures. Additionally, the existing heat transfer fluids exhibit corrosive behaviour, which damage the components of the geothermal well, increasing maintenance costs and reducing system reliability.

Furthermore, some of the heat transfer fluids are not environmentally friendly, posing risks of contamination in case of leaks or spills in the geothermal wells.

Therefore, in light of the foregoing discussion, there exists a need to overcome the aforementioned drawbacks.

### SUMMARY

The aim of the present disclosure is to provide a liquid composition, a geothermal well comprising a liquid composition and a method for preparing a liquid composition to enhance stability, thermal efficiency, and environmental compatibility of the liquid composition. The aim of the present disclosure is achieved by a liquid composition, a geothermal well comprising a liquid composition, and a method for preparing a liquid composition for transferring heat as defined in the appended independent claims to which reference is made to. Advantageous features are set out in the appended dependent claims.

Throughout the description and claims of this specification, the words *"comprise", "include", "have",* and *"contain"* and variations of these words, for example *"comprising"* and *"comprises",* mean *"including but not limited to",* and do not exclude other components, items, integers or steps not explicitly disclosed also to be present. Moreover, the singular encompasses the plural unless the context otherwise requires. In particular, where the indefinite article is used, the specification is to be understood as contemplating plurality as well as singularity, unless the context requires otherwise.

### BRIEF DESCRIPTION OF THE DRAWINGS

FIG. 1 is an illustration of a geothermal well comprising a liquid composition, for transferring heat from the Earth's crust, in accordance with an embodiment of the present disclosure; and
FIG. 2 is an illustration of method for preparing a liquid composition, for transferring heat in a geothermal well, in accordance with an embodiment of the present disclosure.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practicing the present disclosure are also possible.

In a first aspect, the present disclosure provides a liquid composition for transferring heat in a geothermal well, the liquid composition comprising:
ethanol (EtOH); and
potassium formate (KFo);
wherein a mixture of the ethanol (EtOH) and the potassium formate (KFo) is dissolved in a solvent.

The present disclosure seeks to provide an aforementioned liquid composition that is having sub-zero freezing point, which makes the liquid composition operable at sub-zero temperatures. Moreover, the liquid composition provides long-term reliability, due to anti-corrosive properties. Furthermore, the liquid composition is able to enhance the efficiency of absorb the heat from the Earth's crust and transfer the heat to the Earth's surface, ensuring high efficiency and reliability in geothermal applications.

In a second aspect, the present disclosure provides a geothermal well comprising a liquid composition of first aspect, for transferring heat from the Earth's crust, the geothermal well comprising:
a borehole extending from a distal end at or near the Earth's surface to a posterior end positioned at a depth within the Earth's crust; and
a conduit arrangement positioned within the borehole, wherein:
   a first section of the conduit arrangement is configured to transport the liquid composition from the distal end downward towards a heat absorption zone within the Earth's crust, and
   a second section of the conduit arrangement is configured to transport the liquid composition from the heat absorption zone upward towards the distal end of the geothermal well;
wherein the liquid composition is configured to:
   absorb heat from the Earth's crust at the posterior end to heat the liquid composition, and
   transfer the heated liquid to the distal end for utilization in a thermal energy system.

The present disclosure seeks to provide an aforementioned geothermal well that increases an efficiency of transferring the heat from the Earth's crust. Moreover, the use of the liquid composition of the first aspect in the geothermal well, makes the geothermal well operable at sub-zero temperatures, due to sub-zero freezing point of the liquid composition. Furthermore, the use of the liquid composition of the first aspect in the geothermal well enables to increase a long-term reliability of the geothermal well due to anti-corrosive property of the liquid composition. Furthermore, a reduced amount of the ethanol in the liquid composition makes the liquid composition economically viable, non-flammable, environmentally friendly, and operationally safe to use. Furthermore, a low viscosity of the liquid composition reduces pumping costs and an amount of energy consumed by the liquid composition to operate in the geothermal well.

In a third aspect, the present disclosure provides a method for preparing a liquid composition of first aspect, for transferring heat in a geothermal well of second aspect, the method comprising:
mixing ethanol (EtOH) and potassium formate (KFo) in a solvent in a ratio of first value to a second value to a third value, respectively, wherein a sum of the first value, the second value and the third value is equal to 1;
homogenizing the mixture to ensure uniform distribution of the ethanol (EtOH), the potassium formate (KFo) and the solvent in the liquid composition; and
supplying the liquid composition to the geothermal well for transferring heat from earth's crust.

The present disclosure seeks to provide an aforementioned method that increases a homogeneity of the mixture in the liquid composition. Moreover, the increased homogeneity of the mixture in the liquid composition enables the liquid composition to maintain stability and exhibit consistent behavior across an entire volume of the liquid composition.

Throughout the present disclosure, the term *"liquid composition"* refers to a specifically formulated mixture designed for transferring the heat in the geothermal well. In other words, the liquid composition serves as the medium that facilitates the absorption and the transfer of the heat from the Earth's crust to the surface in the geothermal well. Notably, transferring the heat is the primary function of the liquid composition, which is achieved through the circulation of the liquid composition within the geothermal well. Throughout the present disclosure, the term *"geothermal well"* refers to a deep well drilled into the Earth's crust to extract heat from underground reservoirs in the Earth's crust. Notably, the ethanol being present in the liquid composition reduces a freezing point and enhances a heat transfer efficiency of the liquid composition. Similarly, the potassium formate being present in the liquid composition enhances the thermal and physical properties of the liquid composition, such as a density and an anti-corrosive nature of the liquid composition. Also, the combination of ethanol and potassium formate enhances a heat transfer efficiency of the liquid composition, and enhances as well other physical properties and thermal properties.

Throughout the present disclosure, the term *"solvent"* refers to the liquid which acts as a medium for the mixture of the ethanol and the potassium formate to dissolve and form the liquid composition. Notably, the use of the solvent to dissolve the mixture of the ethanol and the potassium formate ensures the uniform distribution of the ethanol and the potassium formate in the liquid composition. Optionally, the solvent is water. Throughout the present disclosure, the phrase *"mixture of the ethanol and the potassium formate"* refers to a uniform and stable blend of the ethanol and the potassium formate in specific proportions. Notably, the mixture of the ethanol and the potassium formate being dissolved in the solvent ensures that the liquid composition achieves the desired thermal and physical properties for transferring the heat in the geothermal well.

Optionally, a ratio of the ethanol to the potassium formate to the solvent is a first value to a second value to a third value, wherein a sum of the first value, the second value and the third value is equal to 1. In this regard, the phrase *"ratio of the ethanol to the potassium formate to the solvent"* refers to a proportional relationship between quantities of the ethanol, the potassium formate, and the solvent in the liquid composition. Notably, the ratio defines the relative amounts of the ethanol, the potassium formate, and the solvent in the liquid composition, ensuring that the liquid composition achieves the desired thermal and physical properties for transferring the heat in the geothermal well. Throughout the present disclosure, the term *"first value"* refers to a numerical value of the relative amount of the ethanol in the ratio of the ethanol to the potassium formate to the solvent. Subsequently, the first value determines the concentration of the ethanol in the liquid composition, which directly influences properties such as freezing point, thermal conductivity, and heat transfer efficiency of the liquid composition. Throughout the present disclosure, the term *"second value"* refers to a numerical value of the relative amount of the potassium formate in the ratio of the ethanol to the potassium formate to the solvent. Subsequently, the second value determines the concentration of potassium formate in the liquid composition, which contributes to properties such as density, anti-corrosive behavior, and thermal stability of the liquid composition. Throughout the present disclosure, the term *"third value"* refers to a numerical value of the relative amount of the solvent in the ratio of the ethanol to the potassium formate to the solvent. Subsequently, the third value determines the concentration of the solvent in the liquid composition, which provides the medium for dissolving the mixture of the ethanol and the potassium formate. A technical effect is that the proportions of the ethanol, the potassium formate, and the solvent in the liquid composition are precisely controlled by defining the ratio of the ethanol to the potassium formate to the solvent as the first value, the second value, and the third value.

Optionally, the first value is in a range of 0.1 to 0.2, the second value is in a range of 0.1 to 0.2, and the third value is in a range of 0.6 to 0.8. Optionally, the first value is in the range of 0.1, 0.12, 0.14, 0.16 and 0.18 to 0.12, 0.14, 0.16, 0.18 and 0.2. Optionally, the second value is in the range of 0.1, 0.12, 0.14, 0.16 and 0.18 to 0.12, 0.14, 0.16, 0.18 and 0.2. Optionally, the third value is in the range of 0.6, 0.64, 0.68, 0.72 and 0.76 to 0.64, 0.68, 0.72, 0.76 and 0.8. A technical effect of the specific ranges for the first value, the second value, and the third value ensures an optimal balance of thermal, physical, and chemical properties is achieved in the liquid composition.

Optionally, the liquid composition comprises a total solute concentration, comprising the ethanol and the potassium formate, of 30 weight percent, wherein the concentration of the ethanol ranges between 10-20 weight percent and the potassium formate ranges between 10-20 weight percent, and wherein the solvent is water maintaining 70 weight percent of the liquid composition. In this regard, the term *"total solute concentration"* refers to a combined weight percentage of all solutes present in the liquid composition, in essence, the ethanol and the potassium formate. The total solute concentration determines the overall concentration of the mixture of the ethanol and the potassium formate in the liquid composition, which directly influences the thermal, physical, and chemical properties of the liquid composition. Subsequently, the total solute concentration, comprising the ethanol and the potassium formate, being 30 weight percent implies that the combined weight percentage of the ethanol and the potassium formate in the liquid composition is 30 percent. Notably, the concentration of the ethanol ranging between 10-20 weight percent provides the specific range for the concentration of the ethanol within the total solute concentration of the liquid composition. Similarly, the concentration of the potassium formate ranging between 10-20 weight percent provides the specific range for the concentration of the potassium formate within the total solute concentration of the liquid composition. Moreover, the solvent being water maintaining 70 weight percent of the liquid composition implies that the water is the solvent used in the liquid composition and constitutes 70% of the total weight of the liquid composition. Subsequently, the solvent being water ensures that the mixture of the ethanol and the potassium formate is effectively dissolved, resulting in the liquid composition being homogeneous and stable. A technical effect is that the weight percentages of the ethanol, the potassium formate, and the solvent in the liquid composition are precisely defined.

Optionally, a viscosity of the liquid composition is a range of 6.8 to 11.2 mPas. In this regard, the phrase *"viscosity of the liquid composition"* refers to a measure of the liquid composition's resistance to flow. Optionally, the viscosity of the liquid composition is in the range of 6.8, 7, 7.5, 8, 8.5, 9, 9.5, 10, 10.5, and 11 mPas to 7, 7.5, 8, 8.5, 9, 9.5, 10, 10.5, 11, and 11.2 mPas. A technical effect of the viscosity of the liquid composition being in the aforementioned range is that the liquid composition is able to maintain stability, and flowability, in the geothermal well.

Optionally, a thermal conductivity of the liquid composition is in a range of 0.427 to 0.452 W/mk. In this regard, the phrase *"thermal conductivity of the liquid composition"* refers to a measure of an ability of the liquid composition to conduct the heat. Subsequently, the thermal conductivity of the liquid composition is the critical thermal property that determines the efficiency with which the liquid composition transfers the heat in the geothermal well. The thermal conductivity of the liquid composition ensures that the liquid composition is able to absorb the heat from the Earth's crust in the geothermal well and transfer the absorbed heat to the surface for utilization. Optionally, the thermal conductivity of the liquid composition is in the range of 0.427, 0.43, 0.435, 0.44, 0.445, and 0.45 W/mk to 0.43, 0.435, 0.44, 0.445, 0.45, and 0.452 W/mk. A technical effect of the thermal conductivity of the liquid composition being in the aforementioned range is that efficient transfer of the heat is ensured in the geothermal well.

Optionally, a specific heat capacity of the liquid composition is in a range of 3.66 to 3.71 kJ/kgK. In this regard, the phrase *"specific heat capacity of the liquid composition"* refers to the amount of heat energy required to raise the temperature of a unit mass of the liquid composition by one degree Kelvin. Subsequently, the specific heat capacity of the liquid composition is a critical thermal property that determines the ability of the liquid composition to store and transfer heat efficiently in the geothermal well. Optionally, the specific heat capacity of the liquid composition is in the range of 3.66, 3.67, 3.68, 3.69, and 3.7 kJ/kgK to 3.67, 3.68, 3.69, 3.7, and 3.71 kJ/kgK. A technical effect of the specific heat capacity of the liquid composition being in the aforementioned range enables to ensure efficient absorption, storage, and transfer of the heat in the geothermal well.

Optionally, a density of the liquid composition is in a range of 1054 to 1067.9 kg/m³. In this regard, the phrase *"density of the liquid composition"* refers to a measure of a mass per unit volume of the liquid composition. Subsequently, the density of the liquid composition is a critical physical property that determines the behavior of the liquid composition during circulation in the geothermal well. Optionally, the density of the liquid composition is in the range of 1054, 1058, 1062, and 1066 kg/m³ to 1058, 1062, 1066, and 1067.9 kg/m³. A technical effect of the density of the liquid composition being in the aforementioned range is that the liquid composition is able to maintain stability, and flowability, in the geothermal well.

Optionally, a freezing point of the liquid composition is in a range of -50 to -15 degrees Celsius. In this regard, the phrase *"freezing point of the liquid composition"* refers to a measure of the temperature at which the liquid composition transitions from a liquid state to a solid state. Subsequently, the freezing point of the liquid composition indicates at what temperature the liquid composition remains in the liquid state under low-temperature conditions encountered during geothermal operations in the geothermal well, thereby maintaining an ability of the liquid composition to circulate and transfer the heat effectively in the geothermal well. Optionally, the freezing point of the liquid composition is in the range of -50, -40, -30, and -20 degrees Celsius to -40, -30, -20, -15 degrees Celsius. A technical effect of the freezing point of the liquid composition being in the aforementioned range is that the liquid composition is suitable to operate in low-temperature environments while remaining in the liquid state.

Optionally, the liquid composition comprises only ethanol (EtOH) and potassium formate (KFo).

Optionally, the liquid composition is biodegradable and anti-corrosive. In this regard, the liquid composition being biodegradable implies to an ability of the liquid composition to decompose into environmentally biodegradable materials in an organic manner. Notably, the liquid composition being biodegradable ensures that the liquid have a minimal environmental impact when released into the environment. Moreover, the liquid composition being anti-corrosive implies to an ability of the liquid composition to prevent or minimize corrosion of the materials that come in contact with the liquid composition, such as a conduit arrangement of the geothermal well. Notably, the liquid composition being anti-corrosive ensures the longevity and structural integrity of the components of the geothermal well in which the liquid composition is used for transferring the heat. A technical effect is that the liquid composition is environmentally friendly and sustainable for use in the geothermal well.

The present disclosure also relates to the geothermal well as described above. Various embodiments and variants disclosed above, with respect to the aforementioned liquid composition, apply mutatis mutandis to the geothermal well.

Throughout the present disclosure, the term *"borehole"* refers to a deep, vertical hole drilled into the ground to reach the Earth's crust. Notably, the borehole provides a pathway in the geothermal well for housing the conduit arrangement. Throughout the present disclosure, the term *"distal end"* refers to a specific end of the borehole, that is positioned at or near the Earth's surface. It will be appreciated that the distal end provides the entry and exit point for the liquid composition in the conduit arrangement positioned within the borehole. Throughout the present disclosure, the term *"depth"* refers to a vertical extent from the Earth's surface to a specified point within the Earth's crust up to which the borehole extends. Notably, the depth within the Earth's crust enables to determine the location of the posterior end of the borehole and the heat absorption zone in the geothermal well. It will be appreciated that the depth within the Earth's crust is selected to be in a region in the Earth's crust with sufficient thermal energy for transferring the heat from the Earth's crust. Throughout the present disclosure, the term *"posterior end"* refers to that end of the borehole located at the lowermost part of the borehole. Notably, the posterior end is located in the borewell at the heat absorption zone within the Earth's crust to enable the conduit arrangement positioned within the borehole to extend to the heat absorption zone within the Earth's crust.

Throughout the present disclosure, the term *"conduit arrangement"* refers to a system of pipes or tubes positioned within the borehole. Notably, the conduit arrangement positioned within the borehole enables the circulation of the liquid composition between the distal end and the posterior end of the borehole. Optionally, the conduit arrangement is in form of a loop starting and ending at the distal end of the borehole. Optionally, the conduit arrangement comprises at least one pipe or tube having a diameter in range of 5 cm to 50 cm. In this regard, the term *"at least one pipe or tube"* refers to one or more cylindrical shaped structural component of the conduit arrangement, that is hollow from the inside to provide a passage for the liquid composition to pass within the conduit arrangement. Notably, the at least one pipe or tube serves as the pathway for the liquid composition to circulate between the distal end and the posterior end of the borehole. Throughout the present disclosure, the term *"diameter"* refers to a width of the cross-sectional area of the at least one pipe or tube. The diameter determines the capacity of the at least one pipe or tube to transport the liquid composition and affect a flow rate and pressure of the liquid composition within the conduit arrangement. Optionally, the diameter of the at least one pipe or tube is in the range of 5, 10, 20, 30, and 40 cm to 10, 20, 30, 40, and 50 cm. A technical effect of the diameter of the at least one pipe or tube being in the aforementioned range is that the conduit arrangement is capable to efficiently transport the liquid composition between the distal end and the posterior end of the borehole.

Optionally, the conduit arrangement comprises two or more separate pipes or tubes. In this regard, the phrase *"two or more separate pipes or tubes"* refers to two or more distinct cylindrical shaped structural components of the conduit arrangement, that is hollow from the inside to provide a passage for the liquid composition to pass within the conduit arrangement. Notably, the two or more separate pipes or tubes are used to enable independent circulation of the liquid composition in distinct flow paths in the conduit arrangement. Optionally, a first pipe or tube from amongst the two or more separate pipes or tubes is used as the first section of the conduit arrangement, and a second pipe or tube from amongst the two or more separate pipes or tubes is used as the second section of the conduit arrangement. A technical effect is that the conduit arrangement's operational efficiency is enhanced through by using the two or more separate pipes or tubes.

Optionally, wherein the conduit arrangement is made from one of: metal material, plastic material, fiber material or a hybrid material. It will be appreciated that using one of the aforementioned materials to make the conduit arrangement is well known in the art. A technical effect of the conduit arrangement being made from one of the aforementioned materials is that the conduit arrangement is reliable, cost efficient and easy to manufacture.

Throughout the present disclosure, the term *"heat absorption zone"* refers to the region within the Earth's crust where the liquid composition absorbs heat. It will be appreciated that the heat absorption zone is situated at the posterior end of the borehole, ensuring that the liquid composition is exposed to sufficient thermal energy for transferring the heat. Throughout the present disclosure, the term *"first section"* refers to that part of the conduit arrangement which transports the liquid composition from the distal end downward towards the heat absorption zone at the posterior end. Notably, the first section being configured to transport the liquid composition from the distal end downward towards the heat absorption zone ensures that the liquid composition is provided with a passage in the conduit arrangement to reach the heat absorption zone at the posterior end from the distal end. Optionally, when the conduit arrangement is in form of a loop, a first half of the conduit arrangement with respect to a vertical axis is used as the first section. Throughout the present disclosure, the term *"second section"* refers to that part of the conduit arrangement which transports the liquid composition from the heat absorption zone at the posterior end upward towards the distal end. Notably, the second section being configured to transport the liquid composition from the heat absorption zone at the posterior end upward towards the distal end ensures that the liquid composition is provided with a passage in the conduit arrangement to reach the distal end from the heat absorption zone.

Notably, when the liquid composition is at the posterior end in the conduit arrangement, the liquid composition comes in contact with the heat in the heat absorption zone, which enables the liquid composition to absorb the heat from the Earth's crust. Subsequently, the heat absorbed from the Earth's crust enables to heat the liquid composition, based on a high thermal conductivity and a high specific heat capacity of the liquid composition.

Subsequently, the heated liquid being transferred to the distal end for utilization in the thermal energy system implies that the liquid composition, after being heated in the heat absorption zone, is transported to the Earth's surface at the distal end for use in the thermal energy system. It will be appreciated that the heated liquid is transferred to the distal end, using the first section of the conduit arrangement. Throughout the present disclosure, the term *"thermal energy system"* refers to a system that utilizes the heat transferred by the liquid composition for various applications, such as power generation or heating. Notably, the thermal energy system enables to effectively utilize the heat absorbed by the liquid composition.

The present disclosure also relates to the method as described above. Various embodiments and variants disclosed above, with respect to the aforementioned liquid composition and the aforementioned geothermal well, apply mutatis mutandis to the method.

Throughout the present disclosure, the term *"uniform distribution"* refers to the even dispersion of the ethanol, the potassium formate, and the solvent throughout the liquid composition. Notably, the uniform distribution of the ethanol, the potassium formate, and the solvent in the liquid composition ensures that the liquid composition achieves consistent thermal, physical, and chemical properties across an entire volume of the liquid composition. Throughout the present disclosure, the phrase *"homogenizing the mixture"* refers to the process of thoroughly mixing the ethanol, the potassium formate, and the solvent to achieve the uniform distribution of aforementioned components within the liquid composition. Subsequently, the homogenizing of the mixture ensures that the liquid composition is stable and consistent without any variations in concentration or properties of the liquid composition.

### DETAILED DESCRIPTION OF THE DRAWINGS

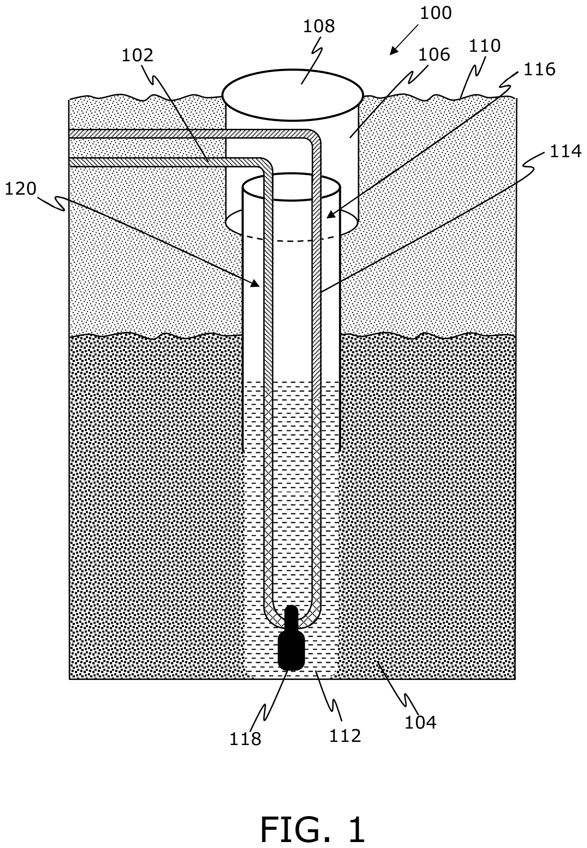

Referring to FIG. 1, illustrated is schematic illustration of a geothermal well **100** comprising a liquid composition **102,** for transferring heat from the Earth's crust **104** in accordance with an embodiment of the present disclosure. The geothermal well **100** comprises a borehole **106** extending from a distal end **108** at or near the Earth's surface **110** to a posterior end **112** positioned at a depth within the Earth's crust **104.** Moreover, the geothermal well comprises a conduit arrangement **114** positioned within the borehole **106,** wherein a first section **116** of the conduit arrangement **114** is configured to transport the liquid composition **102** from the distal end **108** downward towards a heat absorption zone **118** within the Earth's crust **104,** and a second section **120** of the conduit arrangement **114** is configured to transport the liquid composition **102** from the heat absorption zone **118** upward towards the distal end **108** of the geothermal well **100;** wherein the liquid composition **102** is configured to absorb heat from the Earth's crust **104** at the posterior end **112** to heat the liquid composition **102** and transfer the heated liquid to the distal end **108** for utilization in a thermal energy system.

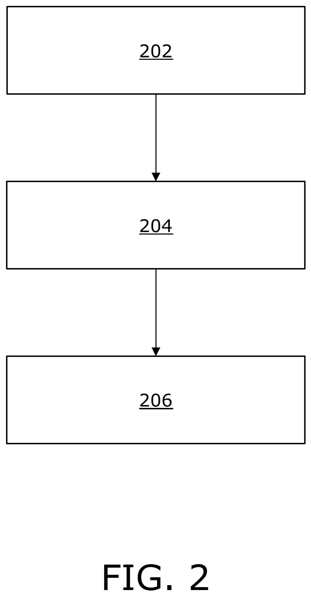

Referring to FIG. 2, illustrated is a method preparing a liquid composition, for transferring heat in a geothermal well, in accordance with an embodiment of the present disclosure. At step **202,** ethanol (EtOH) and potassium formate (KFo) are mixed in a solvent in a ratio of first value to a second value to a third value, respectively, wherein a sum of the first value, the second value and the third value is equal to 1. At step **204,** the mixture is homogenized to ensure uniform distribution of the ethanol (EtOH), the potassium formate (KFo) and the solvent in the liquid composition. At step **206,** the liquid composition is transferred to the geothermal well for transferring heat from earth's crust.

## Claims

1. A liquid composition (102) for transferring heat in a geothermal well (100), the liquid composition comprising:
ethanol (EtOH); and
potassium formate (KFo);
wherein a mixture of the ethanol (EtOH) and the potassium formate (KFo) is dissolved in a solvent.

2. A liquid composition (102) of claim 1, wherein a ratio of the ethanol to the potassium formate to the solvent is a first value to a second value to a third value, wherein a sum of the first value, the second value and the third value is equal to 1.

3. A liquid composition (102) of claim 1 or 2, wherein the first value is in a range of 0.1 to 0.2, the second value is in a range of 0.1 to 0.2, and the third value is in a range of 0.6 to 0.8.

4. A liquid composition (102) of any of the preceding claims, wherein the liquid composition comprises a total solute concentration, comprising the ethanol and the potassium formate, of 30 weight percent, wherein the concentration of the ethanol ranges between 10-20 weight percent and the potassium formate ranges between 10-20 weight percent, and wherein the solvent is water maintaining 70 weight percent of the liquid composition.

5. A liquid composition (102) of any of the preceding claims, wherein a viscosity of the liquid composition is a range of 6.8 to 11.2 mPas.

6. A liquid composition (102) of any of the preceding claims, wherein a thermal conductivity of the liquid composition is in a range of 0.427 to 0.452 W/mk.

7. A liquid composition (102) of any of the preceding claims, wherein a specific heat capacity of the liquid composition is in a range of 3.66 to 3.71 kJ/kgK.

8. A liquid composition (102) of any of the preceding claims, wherein a density of the liquid composition is in a range of 1054 to 1067.9 kg/m3.

9. A liquid composition (102) of any of the preceding claims, wherein a freezing point of the liquid composition is in a range of -50 to -15 degrees Celsius.

10. A liquid composition (102) of any of the preceding claims, wherein the liquid composition is biodegradable and anti-corrosive.

11. A geothermal well (100) comprising a liquid composition (102) of claims 1-10, for transferring heat from the Earth's crust (104), the geothermal well comprising:
a borehole (106) extending from a distal end (108) at or near the Earth's surface (110) to a posterior end (112) positioned at a depth within the Earth's crust; and
a conduit arrangement (114) positioned within the borehole, wherein:
a first section (116) of the conduit arrangement is configured to transport the liquid composition from the distal end downward towards a heat absorption zone (118) within the Earth's crust, and
a second section (120) of the conduit arrangement is configured to transport the liquid composition from the heat absorption zone upward towards the distal end of the geothermal well;
wherein the liquid composition is configured to:
absorb heat from the Earth's crust at the posterior end to heat the liquid composition, and
transfer the heated liquid to the distal end for utilization in a thermal energy system.

12. A geothermal well (100) of claim 11, wherein the conduit arrangement (114) comprises at least one pipe or tube having a diameter in range of 5 cm to 50 cm.

13. A geothermal well (100) of claim 12, wherein the conduit arrangement (114) comprises two or more separate pipes or tubes.

14. A geothermal well (100) of any of the claims 11-13, wherein the conduit arrangement (114) is made from one of: metal material, plastic material, fiber material or a hybrid material.

15. A method for preparing a liquid composition (102) of claims 1-10, for transferring heat in a geothermal well (100) of claims 11-14, the method comprising:
mixing ethanol (EtOH) and potassium formate (KFo) in a solvent in a ratio of first value to a second value to a third value, respectively, wherein a sum of the first value, the second value and the third value is equal to 1;
homogenizing the mixture to ensure uniform distribution of the ethanol (EtOH), the potassium formate (KFo) and the solvent in the liquid composition; and
supplying the liquid composition to the geothermal well for transferring heat from earth's crust (104).

## Amended claims

### Amended claims in accordance with Rule 137(2) EPC.

1. A liquid composition (102) for transferring heat in a geothermal well (100), the liquid composition comprising:
ethanol (EtOH); and
potassium formate (KFo);
wherein a mixture of the ethanol (EtOH) and the potassium formate (KFo) is dissolved in a solvent,
wherein a ratio of the ethanol to the potassium formate to the solvent is a first value to a second value to a third value, wherein a sum of the first value, the second value and the third value is equal to 1, and
wherein the first value is in a range of 0.1 to 0.2, the second value is in a range of 0.1 to 0.2, and the third value is in a range of 0.6 to 0.8.

2. A liquid composition (102) of any of the preceding claims, wherein the liquid composition comprises a total solute concentration, comprising the ethanol and the potassium formate, of 30 weight percent, wherein the concentration of the ethanol ranges between 10-20 weight percent and the potassium formate ranges between 10-20 weight percent, and wherein the solvent is water maintaining 70 weight percent of the liquid composition.

3. A liquid composition (102) of any of the preceding claims, wherein a viscosity of the liquid composition, referring to a measure of the liquid composition's resistance to flow, is in a range of 6.8 to 11.2 mPas.

4. A liquid composition (102) of any of the preceding claims, wherein a thermal conductivity of the liquid composition, referring to a measure of an ability of the liquid composition to conduct heat, is in a range of 0.427 to 0.452 W/mk.

5. A liquid composition (102) of any of the preceding claims, wherein a specific heat capacity of the liquid composition, referring to an amount of heat energy required to raise temperature of a unit mass of the liquid composition by one degree Kelvin, is in a range of 3.66 to 3.71 kJ/kgK.

6. A liquid composition (102) of any of the preceding claims, wherein a density of the liquid composition, referring to a measure of mass per unit volume of the liquid composition, is in a range of 1054 to 1067.9 kg/m3.

7. A liquid composition (102) of any of the preceding claims, wherein a freezing point of the liquid composition, referring to a measure of temperature at which the liquid composition transitions from a liquid state to a solid state, is in a range of -50 to -15 degrees Celsius.

8. A geothermal well (100) comprising a liquid composition (102) of claims 1-7, for transferring heat from the Earth's crust (104), the geothermal well comprising:
a borehole (106) extending from a distal end (108) at or near the Earth's surface (110) to a posterior end (112) positioned at a depth within the Earth's crust; and
a conduit arrangement (114) positioned within the borehole, wherein:
a first section (116) of the conduit arrangement is configured to transport the liquid composition from the distal end downward towards a heat absorption zone (118) within the Earth's crust, and
a second section (120) of the conduit arrangement is configured to transport the liquid composition from the heat absorption zone upward towards the distal end of the geothermal well;
wherein the liquid composition is configured to:
absorb heat from the Earth's crust at the posterior end to heat the liquid composition, and
transfer the heated liquid to the distal end for utilization in a thermal energy system.

9. A geothermal well (100) of claim 8, wherein the conduit arrangement (114) comprises at least one pipe or tube having a diameter in range of 5 cm to 50 cm.

10. A geothermal well (100) of claim 9, wherein the conduit arrangement (114) comprises two or more separate pipes or tubes.

11. A geothermal well (100) of any of the claims 8-10, wherein the conduit arrangement (114) is made from one of: metal material, plastic material, fiber material or a hybrid material.

12. A method for preparing a liquid composition (102) of claims 1-7, for transferring heat in a geothermal well (100) of claims 8-11, the method comprising:
mixing ethanol (EtOH) and potassium formate (KFo) in a solvent in a ratio of first value to a second value to a third value, respectively, wherein a sum of the first value, the second value and the third value is equal to 1, wherein the first value is in a range of 0.1 to 0.2, the second value is in a range of 0.1 to 0.2, and the third value is in a range of 0.6 to 0.8;
homogenizing the mixture to ensure uniform distribution of the ethanol (EtOH), the potassium formate (KFo) and the solvent in the liquid composition; and
supplying the liquid composition to the geothermal well for transferring heat from earth's crust (104).
